# EUROPEAN PATENT SPECIFICATION

(11) **EP 2 598 103 B1**
(45) Date of publication and mention of the grant of the patent: **18.12.2019**
(21) Application number: 11738443.8
(22) Date of filing: 29.07.2011
(51) Int. Cl.: A61K 8/02, A61K 8/97, A61K 8/99, A61Q 19/02, A61Q 19/08

(54) **USE OF ROASTED COFFEE BEANS FOR REGULATING SKIN PIGMENTATION**
VERWENDUNG VON GERÖSTETEN KAFFEEBOHNEN ZUR REGULIERUNG DER HAUTPIGMENTIERUNG
UTILISATION DES GRAINS DE CAFÉ POUR LA REGULATION DE LA PIGMENTATION DE LA PEAU

(30) Priority: 30.07.2010 EP 10171385
(43) Date of publication of application: 05.06.2013
(73) Proprietor: Société des Produits Nestlé S.A., 1800 Vevey (CH); L'Oréal, 75008 Paris (FR)
(72) Inventor: KRAEHENBUEHL, Karin, CH-1926 Fully (CH); MAUROUX, Olivier, CH-1543 Grandcour (CH); MOODYCLIFFE, Angus, CH-1073 Savigny (CH); DIONISI, Fabiola, CH-1066 Epalinges (CH); GUITARD, Marjorie, CH-1073 Savigny (CH)
(74) Representative: Nony
(86) International application number: PCT/EP2011/063063
(87) International publication number: WO 2012/013764

(56) References cited:
- WO-A1-2004/026287
- WO-A2-2005/102373
- US-A1- 2006 018 986
- US-A1- 2009 169 651

## Description

The present invention relates generally to the field of food supplements and/or food products for cosmetic purpose. More specifically, the present invention aims to provide an ingredient containing roasted coffee beans or an alcoholic or aqueous-alcoholic extract thereof, for preventing and/or treating over expression or accumulation of melanin in the skin. The present invention also aims at improving skin tone as well as providing a skin lightening or whitening agent.

### Background of the invention

Skin color is primarily determined by the amount and type of melanin, a brown pigment present in the skin. Lower amounts of melanin result in lighter skin color while higher amounts result in darker skin color. Also, hyper-pigmentation in the skin is caused by the over expression or accumulation of melanin in the skin. As a result, the pathway involved in melanin production has been the target for many inhibitors so as to reduce the levels produced. One of the principal enzymes involved in the melanin pathway is tyrosinase.

The synthesis of melanin is a process under hormonal control, including the melanocyte stimulating hormone (MSH) and adrenocorticotropic hormone (ACTH) peptides that are produced from the precursor proopiomelanocortin. It is stimulated by the DNA damages that are caused by UVB-radiations as well.

Then, exposure to the sun over time can induce many biochemical reactions in the skin, leading to sunburn and tanning, for example. Other consequences of exposure to the sun accumulate over time. These changes can result in the development of age spots and create an uneven, mottled skin tone. Unfortunately, many of the commercially available products in today's market are either only marginally effective, or contain active agents that are unstable and lose their potency when incorporated into a final formula.

The ability to modify the expression of pigment content in the skin, to promote an evenness skin tone or lightening skin tone, is highly desired in today's society. Many people desire to modify their skin tone, to reduce aging spots, etc., or for purely cosmetic reasons.

As a result, efforts to develop effective compositions have focused on agents that inhibit the activity of tyrosinase. For example, a variety of tyrosinase inhibitors, such as hydroquinone, vitamin C, cystein, kojic acid, arbutin and glutathione among others have been proposed in topical compositions. Also, a variety of dermatological compositions have been suggested for improving the appearance of pigment disorders such as that observed in melasma, freckles, and age spots.

Also, the use of skin bleaching compositions is widely expanded. However, they either destroy melanin or inhibit its formation. Many of these contain harsh chemicals such as peroxides, acids or formaldehyde, or thiolated materials. Less stringent therapies have other disadvantages.

Topical retinoid and topical corticosteroids have been suggested as hypo-pigmenting agents, as have laser treatment and chemical peels, but these fall short of desirable responses.

Other compositions suggested the use on the skin of natural materials, which have in some cases been used for centuries in Asia or Europe to bleach skin and skin areas, or enhance the appearance of fair skin. These include the use of lemon, orange, cucumber, ginkgo, carob, rose fruit, geranium herb, cinnamon, sweet marjoram, rosemary, etc...

In order to combat disorders related to abnormal pigment or to lighten skin tone, various compounds which, when applied topically to the skin, are capable of reducing tyrosinase activity and consequently limiting melanin production, have thus been proposed. Unfortunately, the treatments currently available are not entirely satisfactory, in particular in terms of the side effects which are frequently associated therewith, such as irritant side effects with certain topical agents.

It would thus be highly desirable to have alternative preparations that do not have the drawbacks of those described in the prior art. In particular, it would be highly desirable to develop nutritional cosmetic compositions to be administered via oral route that have improved stability and efficacy to promote an evenness skin tone or to lighten skin tone.

There also remains a need to active agents that are effective for treating and/or preventing skin pigmentation disorders, in particular those due to environmental factors or aging.

The object of the present invention is to meet these needs.

### Summary of the invention

The present inventors could achieve this object by providing a food or food supplement composition that comprises at least an ingredient containing roasted coffee beans.

Thus, according to a first subject, the invention relates to the cosmetic use of an effective amount of at least an ingredient containing roasted coffee beans or an alcoholic or aqueous-alcoholic extract thereof, as an active agent for treating and/or preventing over expression or accumulation of melanin in the skin. Such skin disorder are in particular those due to age or to environmental factors (e.g. UV), such as age-spots. It may also be skin disorders that are observed in melasma and freckles.

The present inventors have discovered that roasted coffee beans effectively suppress the formation of melanin, melanogenesis, despite the fact that the extracts show little to no inhibition of tyrosinase activity. This result is surprising and unexpected considering the pivotal role of tyrosinase in melanogenesis and the focus of development efforts in the art to inhibit this enzyme.

For the purpose of the present invention, the term "skin" is intended to mean the skin of the face or of the body.

For the purpose of the present invention the term "effective amount", is intended to mean an amount sufficient to obtain the expected effect.

For the purpose of the present invention the term "prevent" is intended to mean the fact of reducing the risk of occurrence of the manifestation of the disorder under consideration.

The present invention is also directed towards the cosmetic use of the abovementioned ingredient, as an active agent for treating and/or preventing the skin pigment imperfections. As a result, the complexion becomes brighter and more homogeneous, without areas of dyschromia, or of dryness.

The present invention is also directed towards the cosmetic use of an effective amount of at least roasted coffee beans or an alcoholic or aqueous-alcoholic extract thereof, according to the invention, as an active agent for whitening or lightening skin tone.

A use is contemplated that also comprises the use of at least an ingredient containing roasted coffee beans or an alcoholic or aqueous-alcoholic extract thereof, in combination with an effective amount of at least one active agent for further improving skin hydration or skin ageing, in particular as described hereinafter.

According to another of its aspects, the subject of the invention is a method, in particular a cosmetic method, for treating and/or preventing over expression or accumulation of melanin in the skin in particular aesthetic disorders, in an individual, comprising at least one step of administering, to said individual, at least roasted coffee beans or an alcoholic or aqueous-alcoholic extract thereof, according to the invention.

Compositions according to the present invention are orally administrable. This has the advantage of acting globally on the entire skin, in its deep layers (dermis, hypodermis), by means of a rapid and relatively non-restrictive mode of administration. Specifically, the metabolites and other active nutriments are in particular distributed within the dermal matrix by means of the bloodstream. Oral administration also has the advantage of a rapid and relatively non-restrictive mode of administration.

### Detailed description of the invention

According to a first object, the present invention provides the use of roasted coffee beans.

In the text which follows" coffee beans" must be understood to mean the bean obtained by the moist route (fermentation, washing) or by the dry route (drying followed by mechanical husking) starting from the coffee "cherry", after husking as described above.

"Extract" must be understood to mean all of the compounds obtained starting from an alcoholic or aqueous-alcoholic extraction of a crude product, in this instance decaffeinated coffee beans, roasted or unroasted. The species of coffee trees selected for the preparation of the extracts of coffee beans used in the compositions are advantageously selected from the Coffea species.

Coffee trees are small trees with smooth-margined, perennial, coriaceous, glossy leaves (10-15 x 4-6 cm). The white, fragrant flowers are grouped in whorls at the axil of the leaves. The fruit is a green drupe, which becomes red at maturity and usually contains two planar-convex berries which are made contiguous through their planar face. Although only two species supply the essential needs of the coffee market (C. arabica and C. canephora), many species of coffee trees exist in the wild state in the tropical forests of East Africa.

The coffee "bean" is obtained by the moist route (fermentation, washing) or the dry route (drying, followed by mechanical decortication) starting from the coffee"cherry", i.e. from the drupes. The reduction to pulp removes the red pericarp and the fleshy mesocarp; it leads to the coffee "husk". It is after husking (removal of the lignified endocarp) that the coffee "berry" (or bean) is obtained.

The roasted coffee may be a blend of Arabica and Robusta, although it is particularly preferred that it is substantially or wholly derived from Arabica beans. This is because Arabica provides a richer taste and aroma profile, which is associated with coffee of higher quality.

The roasted coffee may have different roasting levels form light to dark, preferably CTN from 60 to 100.

The roasted coffee portion may be at least partially decaffeinated. This is to provide products the caffeine level of which can be modulated from caffeine-free to regular caffeine level.

In particular, a coffee bean extract can be obtained by an aqueous- alcoholic or alcoholic extraction of coffee beans, and preferably by an extraction with the aid of methanol, ethanol or propanol. Preferably, it does not contain the fractions of coffee beans extractable by nonpolar solvents. Methods for the preparation of decaffeinated coffee extracts are well known in the art.

The compositions according to the invention may be in any of the galenical forms normally available for the method of administration selected. The carrier may be of diverse nature depending on the type of composition under consideration. Accordingly, the composition may be any food or pharmaceutical product, or a cosmetic product for oral application. Examples for food or pharmaceutical carriers nutritional complete formula, dairy product such as milk, yogurt, cheese, fermented milks, milk-based fermented products, ice-creams, cereal-based products or fermented cereal-based products, milk-based powders, infant and baby formulas, chilled or shelf stable beverages, food products of confectionary, chocolate or cereal type, animal feed, in particular for domestic animals. In particular, the chicory or extract thereof may be incorporated into any other form of food supplement or enriched food, for example food bars or compacted or non-compacted powders. The powders may be diluted in water, soda, milk products or soya bean derivatives, or be incorporated into food bars.

The food product may also further comprise a protein source, a carbohydrate source, a lipid source, a mineral source and/or a vitamin source. The presence of proteins, carbohydrates, lipids, minerals and/or vitamins may have several advantages. These compounds generally contribute to the taste and mouthfeel of the final product. They also provide the body with nutrients that it may need urgently when it is affected by skin disorders. They also allow formulating the product of the present invention as a complete nutritional formula, so that no additional nutrition is needed.

The individual ingredients may comprise any kind of edible compound. Typical ingredients for food compositions, in particular drinks, are well known in the art, e.g. milk, cream, coffee whiteners, and coffee creamers. Alternatively, ingredients could also be a salad dressing or parts thereof, for example. Such compounds are used by consumers to modify e.g. the aroma, appearance and texture of a composition. The ingredients may be in liquid or dry form, e.g. as powders, that are dissolved and/or suspended in a drink.

The invention also provides a coffee beverage product, such as (a) a roast and ground coffee product, (b) a soluble coffee product, (c) a single portion coffee pad, tablet or capsule (d) a ready to drink coffee product or (e) any other suitable coffee product, comprising the coffee product as defined herein as an ingredient.

Soluble coffee or instant coffee as it is otherwise known, is very well known and has been produced commercially for many decades. It is produced from roast and ground coffee in a variety of ways, all of which are well known to the person skilled in the art.

The roasted coffee bean is particularly suitable for use as a soluble coffee product or any product, which is based on or derived from a soluble coffee product. For instance, the coffee product may also be used in so-called ready-to-drink beverages. Examples of ready-to-drink beverages for which the product of the present invention are suited include two-in-one beverages which comprise a coffee component together with a natural or artificial sweetener component, the components optionally being pre-diluted with a liquid such as water or milk.

In this case, the coffee component can comprise the coffee product of the present invention.

Three-in-one beverages, which comprise coffee, a sweetener and a whitener such as milk, a liquid creamer or a solid (e.g. powdered) creamer, may comprise the coffee product of the present invention.

Furthermore, any beverage, which comprises soluble coffee as an ingredient, may comprise the coffee product of the present invention.

The composition may further comprise further ingredient suitable for inclusion in a food composition. Usual ingredients may e.g. be sugars, artificial sweeteners, emulsifiers, stabilizers, thickeners, flowing agents, colors, flavors, aromas, and the like. Suitable artificial sweeteners include saccharin, cyclamates, acetosulfame, L-aspartyl based sweeteners such as aspartame, and mixtures of these. Suitable emulsifiers include monoglycerides, diglycerides, lecithin, diacetyl tartaric acid esters of mono-diglycerides, emulsifying starches, and mixtures thereof. Suitable stabilisers include dipotassium phosphate and sodium citrate. A suitable flowing agent is sodium silica aluminate. In one embodiment the composition comprises milk protein and/or vegetable protein. In a further embodiment the composition comprises milk fat and/or vegetable fat.

Additionally, the present invention can be used as an ingredient for standard brew, espresso, coffee-based beverage preparations to be used as bulk or in single beverage portion for in-home preparation.

If the product is a nutritional supplement for oral administration it may be present in capsules, gelatin capsules, soft capsules, tablets, sugar-coated tablets, pills, pastes or pastilles, gums, or drinkable solutions or emulsions, a syrup or a gel. Such a supplement might also include a sweetener, a stabilizer, an antioxidant, an additive, a flavoring agent and/or a colorant. The formulation thereof is carried out by means of the usual methods for producing sugar-coated tablets, gel capsules, gels, hydrogels for controlled release, emulsions, tablets or capsules.

In this respect the adjuvant for oral compositions, in particular for dietary supplements, are known to the specialist. Mention may be made, among others and for purely illustrative purposes, of lubricants such as magnesium stearate, products for instantaneous solubilisation, gelling agents, thickeners, moisturizers, fatty and/or aqueous compounds, preservatives, texturizing, flavoring and/or coating agents, anti-oxidants and coloring materials usually used in foods.

The composition may contain, in addition, lipids, polyphenols, taurine, probiotic microorganisms, vitamins and/or oligo-elements. If probiotics are used, they may be included in a live form, semi-active or in a desactivated form, e. g., as a lyophilized powder.

Also culture supernatants of the micro-organisms may be included in the composition. They may be selected from the group consisting of Lactic acid bacteria, in particular Lactobacilli and/or Bifidobacteria and are more preferably selected among the group consisting of Lactobacillus johnsonii, Lactobacilus reuteri, Lactobacillus rhamnosus, Lactobacillus paracasei, Lactobacillus casei, Bifidobacterium bifidum, Bifidobacterium breve, Bifidobacterium animais, Bifidobacterium infantis, Bifidobacterium dolescentis and Bifidobacterium pseudocatenulatum. According to a most preferred embodiment the strains used are Lactobacillus johnsonii (La1) deposited on June 30, 1992, under Budapest Treaty with the Institute Pasteur and receiving the deposit no. CNCM 1-1225 or Lactobacillus paracasei (ST11) deposited on January 12, 1999, with the Institute Pasteur according to the Budapest Treaty and receiving the deposit no. CNCM I- 2116. The following compounds may for example be used alone or in combination: zinc and its salts including zinc sulfate and zinc glucanate, the vitamins B5, B6, B8, C, E or PP, p-carotene and the carotenoids, extracts of garlic in particular in the form of allyl sulfide or oil garlic, selenium, curcumin, the curcuminoids, niacin, lithospermic acid and adenosine. It is understood that the specialist will select such active compounds and, where possible, combine them in such a manner as to improve the effects expected of the composition which is the object of the invention by preventing the desired activity of interest from being inhibited or attenuated.

### Use

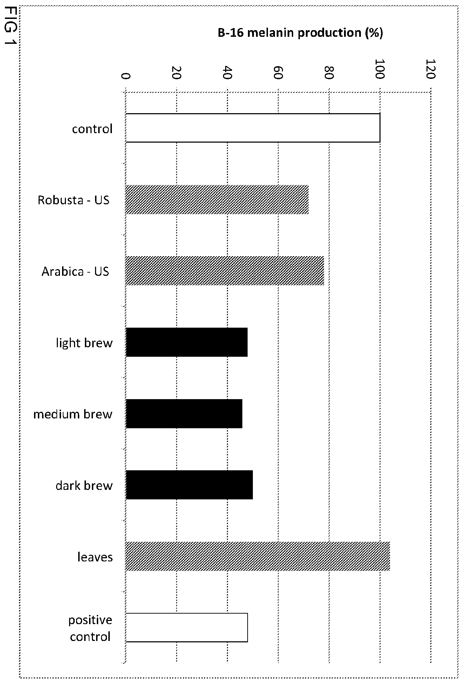

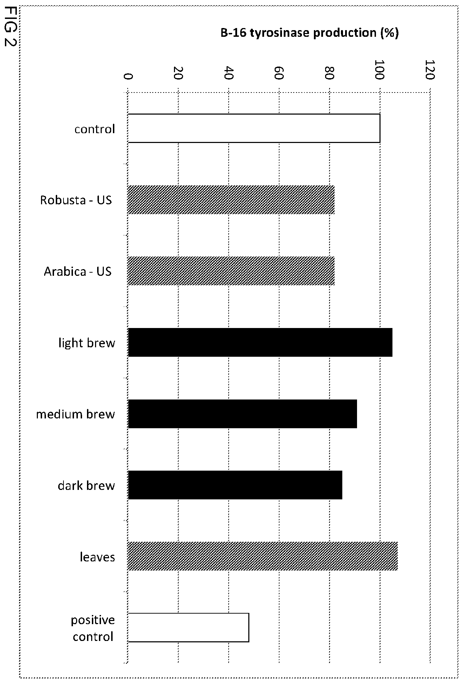

The products are used for treating or preventing skin pigmentation disorders or cosmetically lightening skin tone e.g. by decreasing the production of melanin. Indeed roasted coffee beans extracts were shown to decrease in vitro the synthesis of melanin (Example 1, Figure 1). The production of tyrosinase was also decreased but to a limited extent (Figure 2), suggesting that the decrease in melanin was not due to tyrosinase inhibition but rather to mechanisms acting upstream or downstream of this enzyme.

The ingredients according to the present invention may have further a positive effect on strengthening skin barrier and maintaining skin hydration.

As a result, the pigment imperfections are reduced, the complexion becomes brighter and more homogeneous, without areas of dyschromia, or of dryness.

Thus, according to one subject, the invention relates to the cosmetic use of an effective amount of at least one ingredient containing roasted coffee beans or an alcoholic or aqueous-alcoholic extract thereof, as an active agent for treating and/or preventing over expression or accumulation of melanin in the skin in particular those due to age or environmental factors such as UV.

The present invention is also directed towards the cosmetic use of an effective amount of at least one ingredient containing roasted coffee beans or an alcoholic or aqueous-alcoholic extract thereof, as an active agent for whitening or lightening skin tone, which is particularly desirable for asian population.

A use in accordance with the present invention may also comprises the use of at least one ingredient containing roasted coffee beans or an alcoholic or aqueous-alcoholic extract thereof, in combination with an effective amount of at least one active agent for improving skin hydration or skin ageing, in particular as described hereinafter.

According to another of its aspects, the subject of the invention is a method, in particular a cosmetic method, for treating and/or preventing over expression or accumulation of melanin in the skin in an individual, comprising at least one step of administering, to said individual, at least one ingredient containing roasted coffee beans or an alcoholic or aqueous-alcoholic extract thereof, according to the invention.

The cosmetic treatment method of the invention may be carried out in particular by orally administering at least an effective amount of at least one ingredient containing roasted coffee beans in accordance with the invention. Oral administration comprises ingesting, in one or more intakes, an oral composition as defined above.

It may comprise a single application. According to another embodiment, the application is repeated, for example, 2 to 3 times a day, for one day or more, and generally for a sustained period of at least 4, or even 1 to 15, weeks.

In addition, combinations of treatment with, optionally, oral or topical forms may be envisaged in order to supplement or reinforce the activity of the ingredients as defined by the invention.

Thus, an oral treatment with a composition containing roasted coffee beans in accordance with the invention, combined with a topical composition optionally containing another active ingredient, in particular a probiotic microorganism, or other probiotics in dead, live or semi-active form or an hydrating or anti-ageing agent could be imagined as a kit. The ingredients are mixed, before they are formulated, in the order and under conditions readily determined by those skilled in the art.

The ingredients are mixed, before they are formulated, in the order and under conditions readily determined by those skilled in the art.

The proportion of coffee bean extract in the composition will of course be determined as a function of the desired effect and on the mode of administration of the composition.

The daily doses of roasted coffee bean extract administered by the oral route may preferably be comprised between 0.01 and 5000 mg/day depending on the caffeine content. Preferentially, it is present in the composition according to the invention in a quantity permitting its administration at a dose comprised between 0.5 and 1000 mg/day. The present inventors have found that the effectiveness of roasted coffee bean extracts according to the present invention is generally dose dependant and follows a dose response curve. If generally mild skin disorders or damages are to be prevented and the product will be used frequently, very small amounts will be sufficient to achieve the desired effect. If a severe skin pigment disorder is to be treated, larger amounts will be more appropriate, although also small amounts will produce an effect.

Preferably, the composition intended for oral administration contains an extract of coffee beans in a quantity ranging from 1% to 80% by weight of the composition and preferably from 10 % to 80% by weight of the composition depending on the product form.

Further advantages and features of the present invention are apparent from the following Examples and Figures. The examples hereinafter are thus presented by way of non-limiting illustration of the field of the invention. In these examples, unless otherwise indicated, the percentages are percentages by weight and the ranges of values written in the form "between ... and ..." include the upper and lower limits specified.

### Figures

**Figure 1****:** Assessment of melanin production by murine melanocytes pre-treated with roasted coffee extracts or green coffee extracts.
**Figure 2****:** Assessment of tyrosinase production by murine melanocytes pre-treated with roasted coffee extracts or green coffee extracts.
**Figure 3****:** Assessment of filaggrin synthesis by human primary epidermal keratinocytes pre-treated with different roasted coffee extracts.
**Figure 4****:** Assessment of hyaluronic acid secretion by human primary epidermal keratinocytes pre-treated with coffee extracts.
**Figure 5****:** Production of CD44 by human epidermal keratinocytes pre-treated with different roasted coffee extracts

### EXEMPLES

### Exemple 1 : Effect on skin pigmentation

In order to evaluate the potential beneficial effect of ingredients towards skin de- or pro-pigmentation we used 2D culture of murine melanocytes (B16) and we performed 2 tests: 1-assessment of melanin production and 2- assessment of tyrosinase production.

### 1. The cell culture conditions.

B16 cells were cultured in DMEM 1g/L glucose without phenol red supplemented with 10 % foetal calf serum, in a humidified chamber at 37 °C and containing 5% CO₂.

### 2. The production of melanin by 816 murine melanocyte cell line.

Cells were incubated with the selected ingredients or the test references (Kojic acid at 400µg/mL) for 72 hours, in the presence or absence of NDP-MSH an analog of MSH. The total quantity of melanin (extracellular and intracellular) was evaluated by measurement of the optical density at 405 nm of each sample against melanin standards in presence or in absence of NDP-MSH.

### 3. The production of tyrosinase by 816 murine melanocyte cell line.

Cells were incubated with the selected ingredients or the test references (Kojic acid at 400µg/mL) for 48 hours. The production of tyrosinase was evaluated by immunolabeling.

### INGREDIENTS:

We have tested green coffee extracts of Robusta and Arabica and roasted coffee brews (with three different levels of roasting: light (CTN 100), medium (CTN 80) and dark (CTN 60)).

The tested concentration for each coffee extract is indicated in Table 1.

**Table 1**

| **Ingredient** | **Highest non cytotoxic conc. on HDF (mg/mL)** | **Highest non cytotoxic conc. on HPEK (mg/mL)** | **Tested conc. on HDF (mg/mL)** | **Tested conc. on HPEK (mg/mL)** |
|---|---|---|---|---|
| Green decaf Robusta - US market | 0.2 | 1 | 0.04 | 0.04 |
| Green decaf Arabica - US market | 0.04 | 1 | 0.04 | 0.04 |
| Robusta light roast, brew | 0.04 | 0.2 | 0.04 | 0.04 |
| Robusta medium roast brew | 0.04 | 0.2 | 0.04 | 0.04 |
| Robusta dark roast brew | 0.04 | 0.2 | 0.04 | 0.04 |
| Green coffee leaves | 0.004 | 0.004 | 0.004 | 0.004 |

We have worked with the highest non cytotoxic concentration, with respect to normalize as much as possible the conditions in between the different extracts to further be able to compare them. Thus, coffee extracts have been tested at a concentration of 40µg/mL.

### Results

Results are expressed in percentage relative to the controlTest reference (Kojic acid) induced, as expected, a decrease in melanin content. Figure 1 shows melanin production by B16 melanocytes treated for 72 hours with the selected ingredients.

We have observed overall that coffee extracts had some potential for skin de-pigmentation as they were able to decrease the production of melanin and tyrosinase *in vitro* compared to control conditions (Figures 1 and 2). However roasted coffee brews, appeared to have a greater potential than green coffee extracts. Light roasted coffee brew decreased the melanin content without affecting tyrosinase production (Figure 2), suggesting mechanisms acting upstream or downstream tyrosinase.

### Exemple 2: Effect on skin barrier function and hydration

The potential beneficial effect of the Extracts of Example 1 towards skin barrier function and skin hydration was evaluated using 2D culture of human primary epidermal keratinocytes and we performed 3 tests: 1- assessment of the synthesis of filaggrin, 2- assessment of the secretion of hyaluronic acid and 3- assessment of the production of CD44 (hyaluronic acid receptor) by human primary epidermal keratinocytes.

### The cell culture conditions.

Human epidermal keratinocytes were cultured in control keratinocytes-SFM medium, in a humidified chamber at 37 °C and containing 5% CO2.

### The synthesis of filaggrin by human epidermal keratinocytes.

Cells were incubated with the selected ingredients or the test references (CaCI2 at 1.5mM) for 144 hours. The production of filaggrin by was evaluated by immunolabeling.

### The secretion of hyaluronic acid by human epidermal keratinocytes.

Cells were incubated with the selected ingredients or the test references (retinoic acid at 10⁻⁷M) for 72 hours. The release of hyaluronic acid by human epidermal keratinocytes in supernatants was quantify by ELISA assay.

### The production of CD44 by human epidermal keratinocytes.

Cells were incubated with the selected ingredients or the test references (retinoic acid at 10⁻⁷M) for 72 hours. The production of CD44 by human epidermal keratinocytes was evaluated by immunolabeling.

### Results

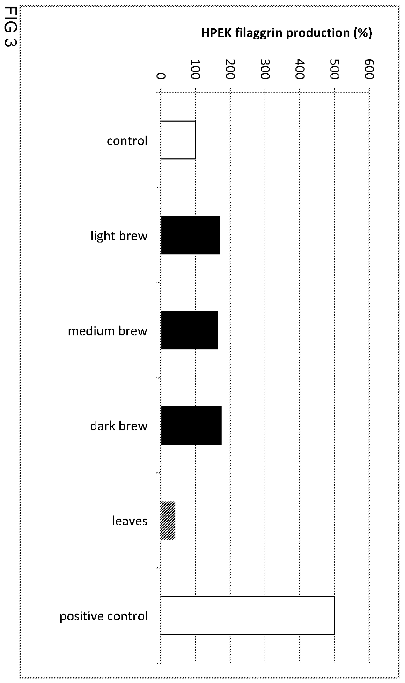

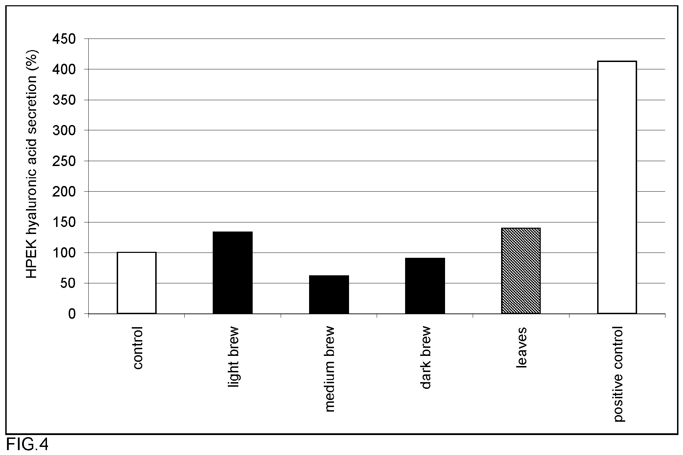

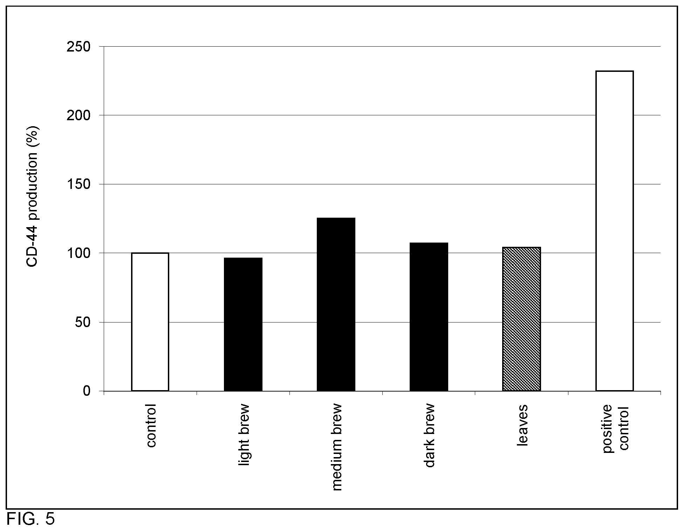

The results are showed in Figure 3, 4 and 5. Pre-treatment of the cells with light, medium or dark roasting coffee brew extracts resulted in an increase of filaggrin of nearly 200%, suggesting that these extracts could strengthen skin barrier (Figure 3). Light coffee brew extracts increased the secretion of hyaluronic acid (133% of the control) (Figure 4), a glycosaminoglycan secreted by skin cells in vivo which help maintain a proper hydration by attracting and trapping water into the extracellular spaces. Whereas medium and dark roasting coffee brews increased moderately (125 % and 107% of the control respectively) the production of CD44, the hyaluronic acid receptor (Figure 5). A stronger skin barrier ensures a better protection of the body from the environment and pathogens' attack. It also limits the loss of water through the epidermis, thus ensuring an appropriate skin hydration.

### Example 3

Preparation of a roasted extract of Coffea robusta 0.5 kg of roasted coffee beans is reduced to a powder by grinding with the Turrax apparatus at 24000 rev/min for 1 minute at 4 C (ice bath).

The powder obtained is mixed with 5 litres of 0.05M phosphate buffer at pH 8.5. The entire mixture is stirred for 30 minutes at 4 C, then centrifuged at 10 000 G at 4 C. The supernatant is filtered through a 0. 22, um filter (sterilizing filtration).

The extract is then fractionated by ultrafiltration through a Sartorius type membrane in order to remove from it oxidation phenomena. The extract is then lyophilized. 29.5 grams of active extract called "lyophilized extract"are thus obtained.

Caffeine is then removed by supercritical chromatography (C02 is used as carrier gas). 25.5 grams of active extract called "decaffeinated lyophilized extract" are thus obtained.

### Example 4: Powder stick

| **Ingredients** | **Amount** |
|---|---|
| **Active ingredient** | |
| Roasted extract as prepared by example 3 | 8 g |

| **Excipient** | |
|---|---|
| Maltodextrin | qs 30 g |
| Xanthan gum | 0.8 mg |
| Sodium benzoate | 0.2 mg |

| | |
|---|---|
| One stick can be taken per day. | |

## Claims

1. Cosmetic, non-therapeutic use of an effective amount of at least roasted coffee beans or an alcoholic or aqueous-alcoholic extract thereof, as an active agent for treating and/or preventing over expression or accumulation of melanin in the skin, wherein said active agent is comprised in an oral composition.

2. Use in accordance with claim 1, **characterized in that** the over expression or accumulation of melanin in the skin is observed in melasma, freckles and/or age spots.

3. Use in accordance with claim 1 or 2 for lightening and/or whitening skin tone.

4. Use in accordance with any one of the preceding claims, in which the extract is derived from coffee beans selected from the species Coffea arabica, Coffea robusta, Coffea canephora or Coffea iberica.

5. Use in accordance with any one of the preceding claims, wherein the coffee beans are decaffeinated.

6. Use in accordance with any one of the preceding claims, in which the coffee bean extract represents from 1% to 80% by weight of the total weight of the composition.

7. Use in accordance with any one of the preceding claims, wherein the roasted coffee bean extract administered by the oral route is comprised between 0.01 and 5000 mg/day, preferably 0.5 and 1000 mg/day.

8. Use according to any one of the preceding claims, in which said composition is a food product, a drink, or a food supplement.

9. Use according to any one of the preceding claims, wherein the composition further comprises at least one kind of food grade micro-organisms, in particular probiotics, in dead, live or semi-active form.

10. Cosmetic, non-therapeutic method for treating and/or preventing over expression or accumulation of melanin in the skin, comprising at least one step of administering, to an individual, an effective amount of at least roasted coffee beans or an alcoholic or aqueous-alcoholic extract thereof, wherein said active agent is comprised in an oral composition.

## Patentansprüche

1. Kosmetische, nichttherapeutische Verwendung einer wirksamen Menge von wenigstens gerösteten Kaffeebohnen oder eines alkoholischen oder wässrigalkoholischen Extraktes davon als Wirkstoff zur Behandlung und/oder Vorbeugung von Überexpression oder Akkumulation von Melanin in der Haut, wobei der Wirkstoff in einer oralen Zusammensetzung enthalten ist.

2. Verwendung gemäß Anspruch 1, **dadurch gekennzeichnet, dass** die Überexpression oder Akkumulation von Melanin in der Haut in Melasma, Sommersprossen und/oder Altersflecken beobachtet wird.

3. Verwendung gemäß Anspruch 1 oder 2 zum Aufhellen und/oder Bleichen der Hautfarbe.

4. Verwendung gemäß einem der vorstehenden Ansprüche, wobei der Extrakt aus Kaffeebohnen ausgewählt aus den Spezies Coffea arabica, Coffea robusta, Coffea canephora und Coffea iberica abgeleitet ist.

5. Verwendung gemäß einem der vorstehenden Ansprüche, wobei die Kaffeebohnen entkoffeiniert sind.

6. Verwendung gemäß einem der vorstehenden Ansprüche, wobei der Kaffeebohnenextrakt von 1 Gew.-% bis 80 Gew.-% des Gesamtgewichts der Zusammensetzung darstellt.

7. Verwendung gemäß einem der vorstehenden Ansprüche, wobei der auf dem oralen Weg verabreichte Extrakt von gerösteten Kaffeebohnen in dem Bereich zwischen 0,01 und 5000 mg/Tag, vorzugsweise 0,5 und 1000 mg/Tag, liegt.

8. Verwendung gemäß einem der vorstehenden Ansprüche, wobei die Zusammensetzung ein Lebensmittelprodukt, ein Getränk oder eine Nahrungsergänzung ist.

9. Verwendung gemäß einem der vorstehenden Ansprüche, wobei die Zusammensetzung ferner wenigstens eine Art von lebensmittelgeeigneten Mikroorganismen, insbesondere Probiotika, in toter, lebender oder halbaktiver Form umfasst.

10. Kosmetisches, nichttherapeutisches Verfahren zur Behandlung und/oder Vorbeugung von Überexpression oder Akkumulation von Melanin in der Haut, umfassend wenigstens einen Schritt der Verabreichung einer wirksamen Menge von wenigstens gerösteten Kaffeebohnen oder eines alkoholischen oder wässrigalkoholischen Extraktes davon, wobei der Wirkstoff in einer oralen Zusammensetzung enthalten ist, an eine Person.

## Revendications

1. Utilisation non thérapeutique cosmétique d'une quantité efficace d'au moins un parmi des grains de café torréfiés ou un extrait alcoolique ou aqueux-alcoolique de ceux-ci, comme agent actif destiné au traitement et/ou à la prévention de la surexpression ou de l'accumulation de mélanine dans la peau, **caractérisée en ce que** ledit agent actif est compris dans une composition orale.

2. Utilisation selon la revendication 1, **caractérisée en ce que** la surexpression ou l'accumulation de mélanine dans la peau est observée dans le mélasme, les taches de rousseur et/ou le lentigo sénile.

3. Utilisation selon la revendication 1 ou 2, destinée à l'éclaircissement et/ou au blanchissement du teint de la peau.

4. Utilisation selon l'une quelconque des revendications précédentes, **caractérisée en ce que** l'extrait est dérivés de grains de café choisis parmi les espèces *Coffea arabica, Coffea robusta, Coffea canephora* ou *Coffea iberica.*

5. Utilisation selon l'une quelconque des revendications précédentes, **caractérisée en ce que** les grains de café sont décaféinés.

6. Utilisation selon l'une quelconque des revendications précédentes, **caractérisée en ce que** l'extrait de grains de café représente de 1% à 80% en poids du poids total de la composition.

7. Utilisation selon l'une quelconque des revendications précédentes, **caractérisée en ce que** l'extrait de grains de café torréfiés administré par voie orale est compris entre 0,01 et 5000 mg/jour, préférablement entre 0,5 et 1000 mg/jour.

8. Utilisation selon l'une quelconque des revendications précédentes, **caractérisée en ce que** ladite composition est un produit alimentaire, une boisson ou un complément alimentaire.

9. Utilisation selon l'une quelconque des revendications précédentes, **caractérisée en ce que** la composition comprend en outre au mois un type de microorganismes de qualité alimentaire, en particulier des probiotiques, sous forme morte, vivante ou semi-active.

10. Méthode non thérapeutique cosmétique destinée au traitement et/ou à la prévention de la surexpression ou de l'accumulation de mélanine dans la peau, comprenant au moins une étape consistant à administrer, à un individu, une quantité efficace d'au moins un parmi des grains de café torréfiés ou un extrait alcoolique ou aqueux-alcoolique de ceux-ci, **caractérisée en ce que** ledit agent actif est compris dans une composition orale.
